Europäisches Patentamt

European Patent Office

Office européen des brevets

(19)

(11) Veröffentlichungsnummer: **0 485 712 B1**

(12) **EUROPÄISCHE PATENTSCHRIFT**

(45) Veröffentlichungstag der Patentschrift: **01.03.95**

(51) Int. Cl.⁶: **C07C 317/22**, C07C 315/04

(21) Anmeldenummer: **91115645.3**

(22) Anmeldetag: **14.09.91**

(54) **Verfahren zur Herstellung von 4,4'-Bis(4-aminophenoxy)diphenylsulfon.**

(30) Priorität: **12.11.90 DE 4035882**

(43) Veröffentlichungstag der Anmeldung:
**20.05.92 Patentblatt 92/21**

(45) Bekanntmachung des Hinweises auf die
Patenterteilung:
**01.03.95 Patentblatt 95/09**

(84) Benannte Vertragsstaaten:
**BE CH DE FR GB IT LI NL**

(56) Entgegenhaltungen:
**EP-A- 0 192 480**
**EP-A- 0 438 665**

(73) Patentinhaber: **HÜLS AKTIENGESELLSCHAFT**

**D-45764 Marl (DE)**

(72) Erfinder: **Poll, Günter, Dr.**
**Venusweg 9**
**W-4370 Marl (DE)**
Erfinder: **Sosna, Friedrich, Dr.**
**Gemener Strasse 35**
**W-4270 Dorsten (DE)**
Erfinder: **Finke, Jürgen, Dr.**
**Münsterlandstrasse 22**
**W-4370 Marl (DE)**

**Beschreibung**

Die Erfindung betrifft ein neues Verfahren zur Herstellung von 4,4'-Bis(4-aminophenoxy)diphenylsulfon aus 4-Aminophenol und 4,4'-Dihalogendiphenylsulfon in einem organischen Lösemittel.

4,4'-Bis(4-aminophenoxy)diphenylsulfon (BAPS) ist ein wichtiges Monomer zur Herstellung von hochschmelzenden Polyamiden, Polyimiden und Polyamidimiden. Es ist darüber hinaus auch als Härter von Epoxiden geeignet.

BAPS wird nach DE-A-19 09 520 aus p-Aminophenol, Natron- oder Kalilauge und einem 4,4'-Dihalogendiphenylsulfon in einem organischen Lösemittel hergestellt. Dieses Verfahren ist zweistufig. Dabei wird zuerst das p-Aminophenolat hergestellt und das dabei entstehende Wasser abdestilliert. Anschließend erhält man durch Umsetzung mit 4,4'-Dichlordiphenylsulfon BAPS. Das Produkt wird mit Nasser gefällt, gelöst, mit Aktivkohle behandelt und erneut gefällt. Es hat dann einen Schmelzpunkt von 191 bis 192 °C, der durch Umkristallisation auf 193 bis 194 °C erhöht werden kann.

Nach DE-A-23 15 607 wird BAPS aus p-Aminophenol, Natronlauge und 4,4'-Dichlordiphenylsulfon hergestellt, wobei stöchiometrische Mengen der Ausgangsverbindungen eingesetzt werden. Dieses Verfahren ist auch zweistufig. In der 1. Stufe wird dabei Wasser mit Hilfe des Schleppmittels Chlorbenzol abdestilliert. Nach zweifacher Fällung wird ein Produkt mit einem Schmelzpunkt von 177 bis 187 °C erhalten. Nach Umkristallisation liegt der Schmelzpunkt bei 188 bis 191 °C.

Auch nach Kawakami et al. (Journal of Polymer Science, Polymer Chemistry Edition, Vol. 12 (1974), 565 - 73) wird BAPS aus p-Aminophenol, Natriumhydroxid und 4,4'-Dichlordiphenylsulfon in Dimethylsulfoxid hergestellt. Man erhält dabei ein Produkt mit einem Schmelzpunkt von 189 bis 191 °C. Nach Umkristallisation liegt der Schmelzpunkt bei 191 bis 192 °C.

Für Polykondensationszwecke wird ein möglichst sauberes BAPS mit einem möglichst hohen Schmelzpunkt benötigt. Dabei ist BAPS mit einem Schmelzpunkt unter 191 °C noch ungeeignet, wohingegen ein Produkt mit einem Schmelzpunkt von 191 bis 193 °C nur zu minderwertigen Polykondensaten mit dunkler Farbe und schlechten mechanischen Eigenschaften führt. Die bekannten Verfahren liefern demnach nur nach mehreren Reinigungsschritten und nach Umkristallisation ein für Polykondensationen gut oder bedingt einsatzbares BAPS.

Aufgabe der vorliegenden Erfindung war es, ein vereinfachtes Verfahren zur Herstellung von BAPS hoher Reinheit zu entwickeln. Dabei sollte nach Möglichkeit bereits nach beendeter Reaktion ein Produkt anfallen, das auch ohne Umkristallisation für Polykondensationszwecke gut geeignet ist.

Diese Aufgabe wird erfindungsgemäß dadurch gelöst, daß man die Reaktion einstufig und in Gegenwart eines anorganischen Carbonats durchführt.

Einstufig im Sinne der Erfindung sind Reaktionen, bei denen Zwischenprodukte weder isoliert noch gefördert werden. Dabei kann man hier beispielsweise so verfahren, daß man 4-Aminophenol, 4,4'-Dihalogendiphenylsulfonund anorganisches Carbonat mischt und dann auf die Reaktionstemperatur erhitzt. Man kann auch 4-Aminophenol und 4,4'-Dihalogendiphenylsulfon erhitzen und dann die Reaktion durch Zugabe von anorganischem Carbonat starten.

Die Reaktion wird vorzugsweise bei 100 bis 250 °C durchgeführt, wobei Temperaturen von 120 bis 200 °C besonders bevorzugt werden. Wenn die Reaktionstemperatur über der Siedetemperatur des eingesetzten Lösemittels liegt, führt man die Reaktion in einer geeigneten Druckapparatur durch.

Die Reaktionszeit liegt üblicherweise im Bereich von 1 bis 200 Stunden. Dabei werden Reaktionszeiten von 1 bis 100 Stunden bevorzugt.

Die Umsetzung von 4-Aminophenol und 4,4'-Dihalogendiphenylsulfon kann in Ethern oder in aliphatischen Halogenkohlenwasserstoffen vorgenommen werden. Vorzugsweise verwendet man jedoch polare, aprotische Lösemittel. Derartige Lösemittel sind beispielsweise Dimethylsulfoxid (DMSO), N-Methylpyrrolidon (NMP), N-Methylcaprolactam, N,N-Dimethylformamid (DMF), N,N-Dimethylacetamid (DMA), Diphenylsulfon, Tetramethylensulfon, Chlorbenzol oder Gemische dieser Lösemittel.

Vorzugsweise führt man die Reaktion in DMSO oder in NMP durch. Die Lösemittelmenge wird so bemessen, daß der Feststoffanteil während der Reaktion 10 bis 80 %, vorzugsweise 20 bis 70 %, beträgt.

Geeignete anorganische Carbonate sind in erster Linie Carbonate und Hydrogencarbonate. Man kann beispielsweise Alkali- und Erdalkalicarbonate wie Lithium-, Natrium-, Kalium-, Magnesium- oder Calciumcarbonat verwenden. Die entsprechenden Hydrogencarbonate können ebenfalls eingesetzt werden. Zinkcarbonat, das basische Zinkhydroxidcarbonat und andere basische Carbonate sind auch geeignet. Vorzugsweise werden jedoch Alkalicarbonate verwendet. Dabei werden Natrium- und Kaliumcarbonat ganz besonders bevorzugt.

Als 4,4'-Dihalogendiphenylsulfon kommen vor allem die Difluor-, die Dichlor- und die Dibromverbindung sowie die Bromchlor-, die Bromfluor- und die Chlorfluorverbindung in Frage. Dabei wird 4,4'-Dichlordiphenylsulfon vorzugsweise eingesetzt.

Das Molverhältnis von 4,4'-Dihalogendiphenylsulfon zu 4-Aminophenol liegt vorzugsweise im Bereich von 1 : 2 bis 1 : 3. Dabei wird ein Molverhältnis von 1 : 2,01 bis 1 : 2,4 besonders bevorzugt.

4,4'-Dihalogendiphenylsulfon und anorganisches Carbonat werden im allgemeinen im Molverhältnis von 1 : 1 bis 1 : 20 eingesetzt. Wird als anorganisches Carbonat ein Alkalicarbonat eingesetzt, so liegt das Molverhältnis vorzugsweise bei 1 : 1 bis 1 : 10. In dem speziellen Fall, daß 4,4'-Dichlordiphenylsulfon und Alkalicarbonat eingesetzt werden, liegt das Molverhältnis vorzugsweise im Bereich von 1 : 1 bis 1 : 5.

Durch das erfindungsgemäße Verfahren wird bei geringem technischen Aufwand in einstufiger Reaktionsführung ein reines BAPS mit einem hohen Schmelzpunkt von meist über 194 °C erhalten. Das Produkt kann auch ohne Umkristallisation direkt für Polykondensationen eingesetzt werden.

In den nachfolgenden Beispielen wird allgemein so verfahren, daß man das 4,4'-Dihalogendiphenylsulfon, 4-Aminophenol und das anorganische Carbonat in einem organischen Lösemittel mischt und dann die Mischung auf die Reaktionstemperatur erhitzt. Nach der Reaktion wird abgekühlt, worauf man BAPS mit Hilfe einer Methanol/Wasser-Mischung ausfällt.

Vor der Fällung kann man das als Nebenprodukt entstandene anorganische Halogenid sowie überschüssiges Carbonat abfiltrieren. Die Fällung von BAPS kann auch durch Zugabe von Alkohol oder von Wasser erfolgen.

Beispiel 1

Alle Arbeiten werden unter Stickstoffabdeckung durchgeführt.

In einem 1-l-Dreihalskolben mit Rührer, Rückflußkühler und Stickstoffeinleitungsrohr werden bei Raumtemperatur 50,25 g (0,175 mol) 4,4'-Dichlordiphenylsulfon, 40,11 g (0,368 mol) 4-Aminophenol, 55,10 g (0,193 mol) $Na_2CO_3 \cdot 10\ H_2O$ in 200 ml NMP vorgelegt. Diese Mischung wird im Verlauf einer Stunde auf 180 °C aufgeheizt und anschließend 15 h bei 180 °C gehalten. Die Temperatur wird dann auf 60 °C gesenkt. 500 ml Methanol/Wasser-Mischung (1 : 1) werden zugegeben. Dabei fällt das entstandene BAPS aus. Das Produkt wird mit Methanol/Wasser-Mischung gewaschen und dann getrocknet.

| Ausbeute: | 64,8 g (86 % d. Th.) |
|---|---|
| $T_m$: | 195,0 °C (nach DSC) |

Beispiele 2 bis 10

Man verfährt wie in Beispiel 1. Es werden jedoch die in Tabelle 1 angegebenen Änderungen vorgenommen.

Tabelle 1

| Beispiel | DCDPS[1]) (mol) | p-Aminophenol (mol) | anorg. Carbonat | (mol) | Lösemittel | Reaktion Zeit (h) | Reaktion Temp. (°C) | Ausbeute (g) | Ausbeute (%) | $T_m$ (°C) |
|---|---|---|---|---|---|---|---|---|---|---|
| 2 | 0,175 | 0,375 | $Na_2CO_3$ | 0,35 | NMP | 15 | 170 | 61,9 | 82 | 194,8 |
| 3 | 0,175 | 0,375 | $NaHCO_3$ | 0,4 | NMP | 24 | 180 | 60,8 | 81 | 194,3 |
| 4 | 0,175 | 0,40 | $Na_2CO_3$ | 0,20 | DMSO | 24 | 170 | 58,0 | 77 | 194,1 |
| 5 | 0,175 | 0,375 | $MgCO_3$ | 0,20 | NMP | 15 | 180 | 60,5 | 81 | 194,6 |
| 6 | 0,175 | 0,375 | $CaCO_3$ | 0,20 | NMP | 15 | 180 | 58,9 | 78 | 194,8 |
| 7 | 0,175 | 0,525 | $Na_2CO_3$ | 0,25 | NMP | 15 | 180 | 63,4 | 84 | 195,2 |
| 8 | 0,175 | 0,375 | $K_2CO_3$ | 0,20 | NMP | 6 | 170 | 65,6 | 87 | 194,7 |
| 9 | 0,175 | 0,375 | $K_2CO_3$ | 0,25 | NMP | 8 | 160 | 67,2 | 89 | 195,2 |
| 10 | 0,175 | 0,375 | $K_2CO_3$ | 0,35 | NMP | 8 | 160 | 63,4 | 84 | 195,1 |

1) DCDPS: 4,4'-Dichlordiphenylsulfon

**Patentansprüche**

1. Verfahren zur Herstellung von 4,4'-Bis(4-aminophenoxy)diphenylsulfon aus 4-Aminophenol und einem 4,4'-Dihalogendiphenylsulfon in einem organischen Lösemittel,
dadurch gekennzeichnet,
daß man die Reaktion einstufig in Gegenwart eines anorganischen Carbonats durchführt.

4

**2.** Verfahren nach Anspruch 1,
dadurch gekennzeichnet,
daß man die Reaktion bei 100 bis 250 °C ausführt.

**3.** Verfahren nach Anspruch 1,
dadurch gekennzeichnet,
daß man ein polares, aprotisches Lösemittel verwendet.

**4.** Verfahren nach Anspruch 3,
dadurch gekennzeichnet,
daß man die Reaktion in Dimethylsulfoxid oder N-Methylpyrrolidon durchführt.

**5.** Verfahren nach Anspruch 1,
dadurch gekennzeichnet,
daß man die Reaktion in Gegenwart eines Alkalicarbonats durchführt.

**6.** Verfahren nach Anspruch 1,
dadurch gekennzeichnet,
daß man 4,4'-Dichlordiphenylsulfon einsetzt.

**7.** Verfahren nach Anspruch 1,
dadurch gekennzeichnet,
daß man 4,4'-Dihalogendiphenylsulfon und 4-Aminophenol im Molverhältnis 1 : 2 bis 1 : 3 umsetzt.

**8.** Verfahren nach Anspruch 7,
dadurch gekennzeichnet,
daß das Molverhältnis 1 : 2,01 bis 1 : 2,4 beträgt.

**9.** Verfahren nach Anspruch 1,
dadurch gekennzeichnet,
daß man 4,4'-Dihalogendiphenylsulfon und anorganisches Carbonat im Molverhältnis 1 : 1 bis 1 : 20 einsetzt.

**10.** Verfahren nach Anspruch 9,
dadurch gekennzeichnet,
daß man 4,4'-Dihalogendiphenylsulfon und Alkalicarbonat im Molverhältnis 1 : 1 bis 1 : 10 verwendet.

**Claims**

**1.** A process for the preparation of 4,4'-bis(4-aminophenoxy)diphenylsulphone from 4-aminophenol and a 4,4'-dihalodiphenylsulphone in an organic solvent, characterised in that the reaction is carried out in a single stage in the presence of an inorganic carbonate.

**2.** A process according to claim 1, characterised in that the reaction is carried out at 100 to 250°C.

**3.** A process according to claim 1, characterised in that a polar aprotic solvent is used.

**4.** A process according to claim 3, characterised in that the reaction is carried out in dimethylsulphoxide or N-methylpyrrolidone.

**5.** A process according to claim 1, characterised in that the reaction is carried out in the presence of an alkali metal carbonate.

**6.** A process according to claim 1, characterised in that the 4,4'-dichlorodiphenylsulphone is reacted.

**7.** A process according to claim 1, characterised in that the 4,4'-dihalodiphenylsulphone and 4-aminophenol are employed in a molar ratio of 1:2 to 1:3.

8. A process according to claim 7, characterised in that the molar ratio is 1:2.01 to 1:2.4.

9. A process according to claim 1, characterised in that the 4,4'-dihalodiphenylsulphone and inorganic carbonate are employed in a molar ratio of 1:1 to 1:20.

10. A process according to claim 9, characterised in that the 4,4'-dihalodiphenylsulphone and inorganic carbonate are used in a molar ratio of 1:1 to 1:10.

**Revendications**

1. Procédé de préparation de la 4,4'-bis-(4-amino-phénoxy)-diphényl-sulfone à partir de 4-amino-phénol et d'une 4,4'-dihalogéno-diphényl-sulfone dans un solvant organique,
caractérisé en ce que l'on effectue la réaction en une seule étape en présence d'un carbonate minéral.

2. Procédé selon la revendication 1,
caractérisé en ce que l'on effectue la réaction à une température de 100 à 250 °C.

3. Procédé selon la revendication 1, caractérisé en ce que l'on utilise un solvant polaire apro
tique.

4. Procédé selon la revendication 3,
caractérisé en ce que l'on effectue la réaction dans du sulfoxyde de diméthyle ou de la N-méthyl-pyrrolidone.

5. Procédé selon la revendication 1,
caractérisé en ce que l'on effectue la réaction en présence
d'un carbonate alcalin.

6. Procédé selon la revendication 1,
caractérisé en ce que l'on utilise de la 4,4'-dichloro-diphényl-sulfone.

7. Procédé selon la revendication 1,
caractérisé en ce que l'on fait réagir la 4,4'-dihalogéno-diphényl-sulfoneet le 4-amino-phénol dans une proportion molaire de 1 : 2 à 1 : 3.

8. Procédé selon la revendication 7,
caractérisé en ce que la proportion molaire est de 1 : 2,01 à 1 : 2,4.

9. Procédé selon la revendication 1,
caractérisé en ce que l'on utilise la 4,4'-dihalogéno-diphényl-sulfone et le carbonate minéral dans une proportion molaire de 1 : 1 à 1 : 20.

10. Procédé selon la revendication 9,
caractérisé en ce que l'on utilise la 4,4'-dihalogéno-diphényl-sulfone et le carbonate alcalin dans une proportion molaire de 1 : 1 à 1 : 10.